# FASCICULE DE BREVET EUROPEEN

(11) **EP 0 913 134 B1**
(45) Date de publication et mention de la délivrance du brevet: **26.03.2003**
(21) Numéro de dépôt: 98420183.0
(22) Date de dépôt: 13.10.1998
(51) Int. Cl.: A61F 2/38

(54) **Prothèse de genou**
Knieprothese
Knee prostheseis

(30) Priorité: 14.10.1997 FR 9713073
(43) Date de publication de la demande: 06.05.1999
(73) Titulaire: Tornier SA, 38330 Saint Ismier (FR)
(72) Inventeur: Timoteo, Michel, 38410 Saint Martin D'Uriage (FR)
(74) Mandataire: Schmitt, John

(56) Documents cités:
- WO-A-95/25484
- FR-A- 2 663 536
- FR-A- 2 719 466
- US-A- 5 271 747

## Description

L'invention concerne une prothèse de genou. Plus précisément, elle se rapporte à une prothèse de genou du type de celle comprenant un plateau tibial ancré au niveau de l'extrémité supérieure du tibia, le plateau tibial présentant un insert rapporté destiné à coopérer avec une pièce fémorale ancrée dans l'extrémité inférieure du fémur.

Il est connu que l'insert, réalisé la plupart du temps en polyéthylène, est soumis aux contraintes répétées, exercées simultanément par le plateau tibial et la pièce fémorale lors du mouvement d'extension ou de flexion du fémur sur le tibia, ou inversement, les contraintes permanentes entraînant l'usure progressive du polyéthylène.

Pour résoudre ce problème, on a proposé dans le document FR-A-2 735 017, une prothèse du type de celle de l'invention comprenant un pion médian faisant saillie par rapport à la face supérieure de l'élément tibial, ledit pion étant engagé dans une cavité correspondante ménagée sur la face inférieure de l'insert et ce, en combinaison avec des agencements sous forme de plots latéraux réalisés sur la partie supérieure du plateau tibial susceptible de glisser le long de deux décrochements latéraux correspondants, prévus sur la face inférieure de l'insert.

Même si cette configuration permet, lors de la flexion du genou, d'obtenir la rotation de la pièce fémorale sur le plateau tibial, l'insert ne reproduit pas pour autant simultanément le mouvement naturel du ménisque dans le sens postéro-antérieur.

De même, l'insert ne reproduit pas le mouvement du ménisque sur le plateau tibial lors de l'extension du genou, à savoir une translation dans le sens antéro-postérieur du ménisque puis un blocage en rotation de celui-ci.

Pour reproduire l'articulation naturelle du genou, on a proposé dans le document WO-96/38103, une prothèse selon laquelle le plateau tibial présente deux plots en saillie, respectivement antérieur et postérieur, destinés à coopérer respectivement avec deux échancrures ménagées sur la face inférieure de la pièce intermédiaire ou insert.

La prothèse proposée dans ce document, de par la forme des échancrures, permet d'assurer un mouvement à la fois latéro-médian, antéro-postérieur et rotatif de l'insert sur le plateau tibial. Elle présente néanmoins un certain nombre d'inconvénients dans la mesure où la rotation de l'insert lors de la flexion du genou est réalisée selon un axe vertical passant par le plot postérieur du plateau tibial. En d'autres termes, en aucun cas, on ne reproduit ici le mouvement naturel de l'articulation du genou en flexion, selon laquelle la rotation du ménisque sur le plateau tibial est réalisée selon un axe sensiblement central.

De plus et comme précédemment, les agencements prévus au niveau du plateau tibial et de l'insert ne permettent pas d'assurer le blocage en rotation de l'insert lors de l'extension du genou.

On connaît de la demande de brevet en France FR 2663536 (9008190) une prothèse de genou du type à glissement comportant un premier élément métallique fixé à l'extrémité inférieure du fémur, un second élément métallique fixé à l'extrémité supérieure du tibia, et un élément intermédiaire en matière plastique dont les surfaces en contact avec les premier et second éléments sont établies de manière complémentaire.

On constate que l'élément intermédiaire, lors d'un mouvement de flexion du genou, se déplace en translation antéro-postérieur, permettant d'éloigner l'entaille de l'élément intermédiaire du fond de l'échancrure du second élément tibial, afin de permettre une rotation limité de l'élément intermédiaire. Cette rotation est limitée par les cotés de l'entaille qui viennent prendre appui sur le coté de l'échancrure du second élément tibial.

L'invention vise donc à proposer une prothèse de genou permettant de limiter au maximum l'usure de l'insert soumis aux forces de compression et de torsion, aussi bien de la pièce fémorale que de la pièce tibiale, tout en permettant de se rapprocher du fonctionnement naturel de l'articulation du genou.

Pour ce faire, l'invention propose une prothèse du type de celle comprenant un plateau tibial ancré au niveau de l'extrémité supérieure du tibia, et sur lequel est rapporté un insert destiné à coopérer avec une pièce fémorale ancrée à l'extrémité inférieure du fémur.

Cette prothèse de genou se caractérise en ce que le plateau tibial et l'insert présentent des moyens complémentaires aptes à assurer :
- En position d'extension, le blocage en rotation dudit insert sur le plateau tibial ;
- Et, lors d'un mouvement de flexion, d'abord la translation dudit insert dans la direction antéro-postérieur et dans le sens postéro-antérieur, puis la rotation limitée dudit insert sur le plateau tibial.

En d'autres termes, l'invention propose une prothèse de genou dont le plateau tibial et l'insert sont agencés de sorte à permettre, lors de l'extension du genou, le blocage en rotation de l'insert sur le plateau tibial en limitant ainsi l'usure de l'insert par blocage de son mouvement antéro-postérieur et rotatif. De même, les agencements permettent d'assurer une rotation limitée de l'insert sur le plateau tibial à partir d'un certain degré de flexion du tibia sur le fémur, et ce dans le double but de limiter l'usure de l'insert et de reproduire le fonctionnement naturel de l'articulation du genou.

Pour stopper la rotation en extension, tout en limitant ladite rotation en flexion, les moyens complémentaires sont constitués :
- D'une part, d'un bossage ménagé sur la face inférieure de l'insert, sensiblement en position centrale, destiné à coopérer avec un orifice ménagé sur la face supérieure du plateau tibial ;
- Et d'autre part, d'une échancrure réalisée au sein de la face inférieure de l'insert, au niveau de sa bordure postérieure, selon un axe médian antéro-postérieur, de manière à coopérer avec un épaulement en saillie ménagé sur la face supérieure du plateau tibial.

Pour permettre, lors de la flexion du tibia sur le fémur, d'abord la translation dans le sens postéro-antérieur, puis la rotation de l'insert sur le plateau tibial, le bossage se présente sous le forme d'une portion de cylindre et l'orifice est constitué d'une partie postérieure parallélépipédique et d'une partie antérieure hémi-cylindrique de diamètre sensiblement égal à celui du bossage.

Cette forme de réalisation permet en effet d'assurer un mouvement de translation postéro-antérieur de même qu'un mouvement de rotation, tout en interdisant tout mouvement latéral de l'insert sur le plateau tibial.

Pour permettre de limiter la rotation de l'insert sur le plateau tibial lors de la flexion du tibia sur le fémur, l'épaulement en forme de saillie présente la forme générale d'un U, dont les deux branches s'évasent en direction de la bordure postérieure du plateau tibial, l'épaulement étant destiné à coopérer avec une échancrure de même forme générale, ménagée sur la face inférieure de l'insert, dont l'épaisseur et la taille selon l'axe médian antéro-postérieur sont sensiblement égales à celles de l'épaulement, mais dont l'écartement des branches est supérieur.

Selon une première forme de réalisation de l'invention destinée à la mise en oeuvre de prothèses du genou postéro-stabilisées, l'épaulement en saillie et l'échancrure complémentaire sont dépourvus de toute lumière.

En revanche, selon une autre forme de réalisation de l'invention, plus particulièrement destinée à la mise en oeuvre de prothèses du genou avec conservation du ligament croisé postérieur, l'épaulement en saillie et l'échancrure sont partiellement ajourés.

Par ailleurs, selon une autre caractéristique de l'invention, l'écartement des branches de l'échancrure est déterminé pour permettre, lors d'une flexion du tibia sur le fémur d'au moins 30°, de limiter la rotation de l'insert sur le plateau tibial à plus ou moins 15° par rapport à la position neutre de l'insert sur le plateau tibial.

La manière de réaliser l'invention, le fonctionnement, ainsi que les avantages qui découlent de l'invention, ressortiront mieux des exemples de réalisation suivants, à l'appui des figures annexées.

La figure 1 est une représentation schématique en perspective de l'insert et du plateau tibial selon un premier mode de réalisation de l'invention, c'est à dire plus particulièrement destiné à la mise en place d'une prothèse du genou postéro-stabilisée.

La figure 2 est une représentation schématique vue de dessus du plateau tibial selon la figure 1.

La figure 3 est une vue de dessous de l'insert selon la figure 1.

La figure 4 est une vue de dessus de l'ensemble plateau tibial/insert en position d'extension du genou selon la figure 1.

La figure 5a est une vue de dessus de l'ensemble plateau tibial/insert en position de flexion avant rotation du tibia sur le fémur selon la figure 2.

La figure 5b est une vue de dessus de l'ensemble plateau tibial/insert en position de flexion après rotation de l'insert sur le plateau tibial.

La figure 6 est une représentation schématique en perspective d'un autre mode de réalisation de l'invention, mettant en oeuvre une prothèse du genou avec conservation du ligament croisé postérieur du genou.

Les figures 7 à 10 sont des vues identiques aux figures 2 à 5 selon le mode de réalisation de la figure 6.

Les figures 11 et 12 sont des représentations schématiques en section, respectivement transversale et longitudinale du plateau tibial conforme à l'invention.

Les figures 13 et 14 sont des représentations schématiques en section du plateau tibial muni de l'insert conforme à l'invention, respectivement en extension et en flexion.

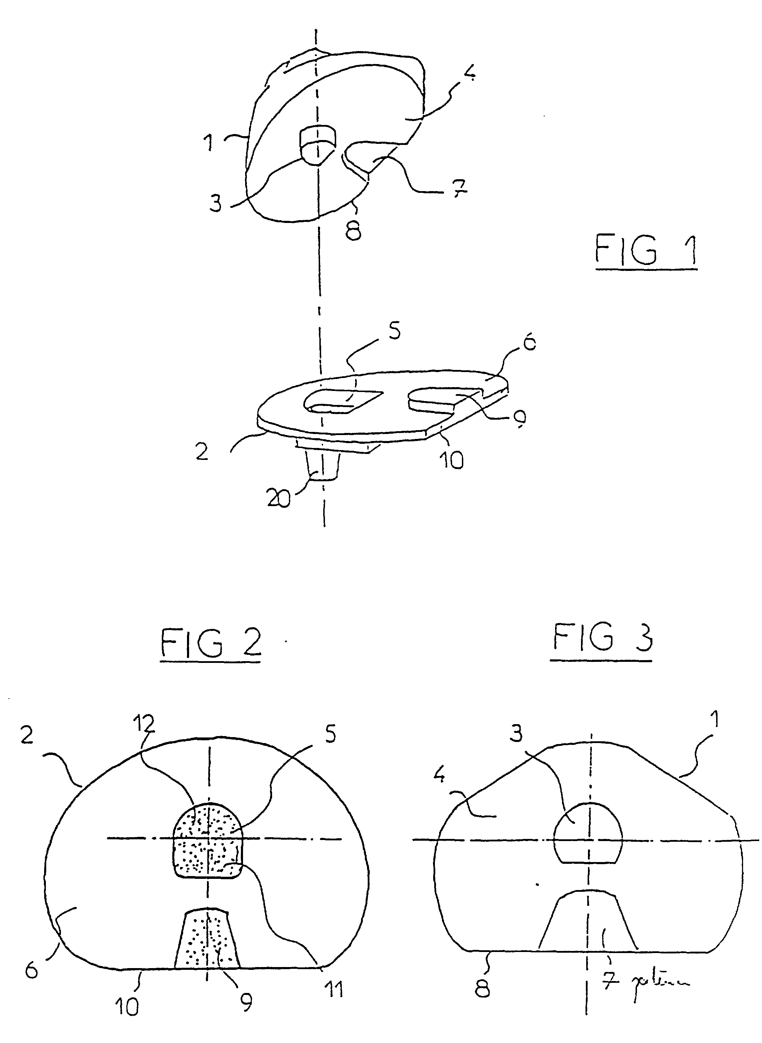

On a représenté sur la figure 1 un insert désigné par la référence générale (1) destiné à être rapporté sur un plateau tibial (2), ledit plateau tibial étant implanté au niveau de l'extrémité supérieure du tibia, et ce, dans le cadre plus général de l'implantation d'une prothèse du genou postéro-stabilisée.

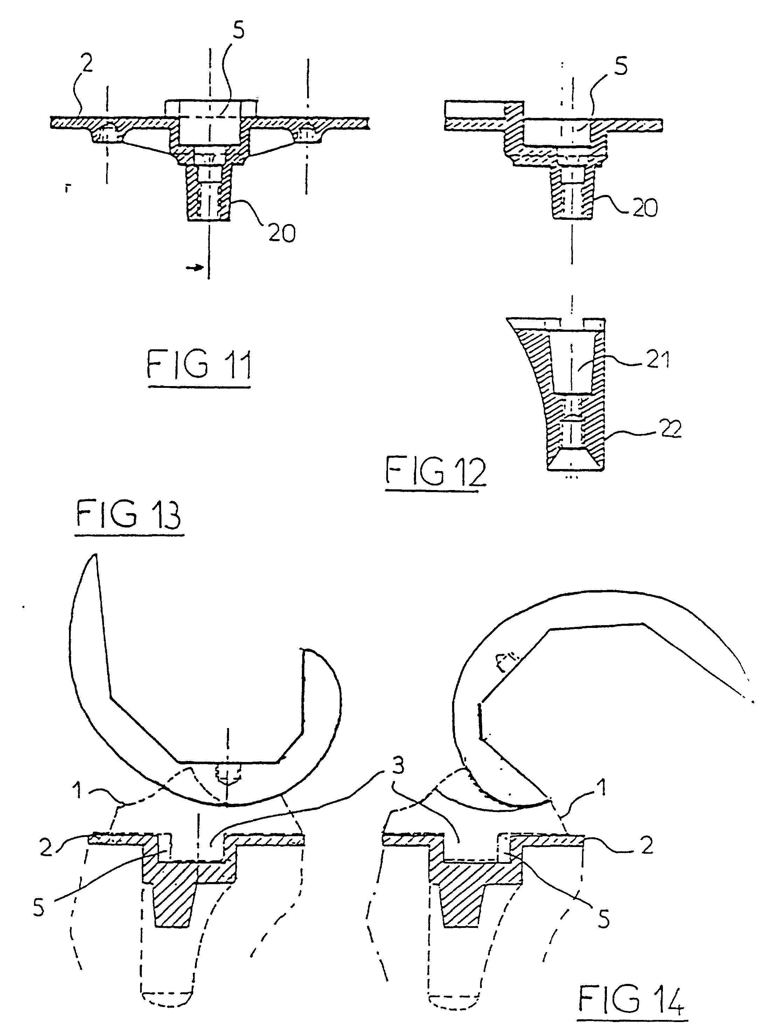

L'insert et le plateau tibial présentent une forme générale connue de l'homme du métier, qui ne fait pas ici l'objet de l'invention. Cependant, ainsi qu'on peut l'observer sur les figures 11 et 12, le plateau tibial (2) présente un orifice central borgne ou non (5), destiné à recevoir le bossage (3) de l'insert, tel que décrit ci-après. Par ailleurs, cet orifice se prolonge par une excroissance (20), en forme de cône morse, destinée à être reçue dans un orifice de forme complémentaire (21), ménagé au sein du plot tibial (22), lui-même inséré dans le canal médullaire du tibia.

L'insert (1) est muni, comme déjà dit d'un bossage (3), ménagé sur sa face inférieure (4) en position centrale et destinée à coopérer avec l'orifice (5) ménagé sur la face supérieure (6) du plateau tibial (2).

L'insert (1) présente en outre une échancrure (7) réalisée au sein de la face inférieure (4) au niveau de la bordure postérieure (8) selon un axe médian antéro-postérieur, ladite échancrure (7) étant destinée à coopérer avec un épaulement (9) réalisé sur la face supérieure de la pièce tibiale (2) au niveau de la bordure postérieure (10).

Selon la première forme de réalisation représentée en figures 1 à 5, le bossage (3) réalisé sur la partie centrale de la face inférieure de l'insert, se présente sous la forme d'une portion de cylindre.

Cette portion de cylindre (3) est destinée à coopérer avec l'orifice (5), lequel présente une partie postérieure parallélépipédique (11) et une partie antérieure hémi-cylindrique (12) de diamètre sensiblement égal à celui de la portion de cylindre (3).

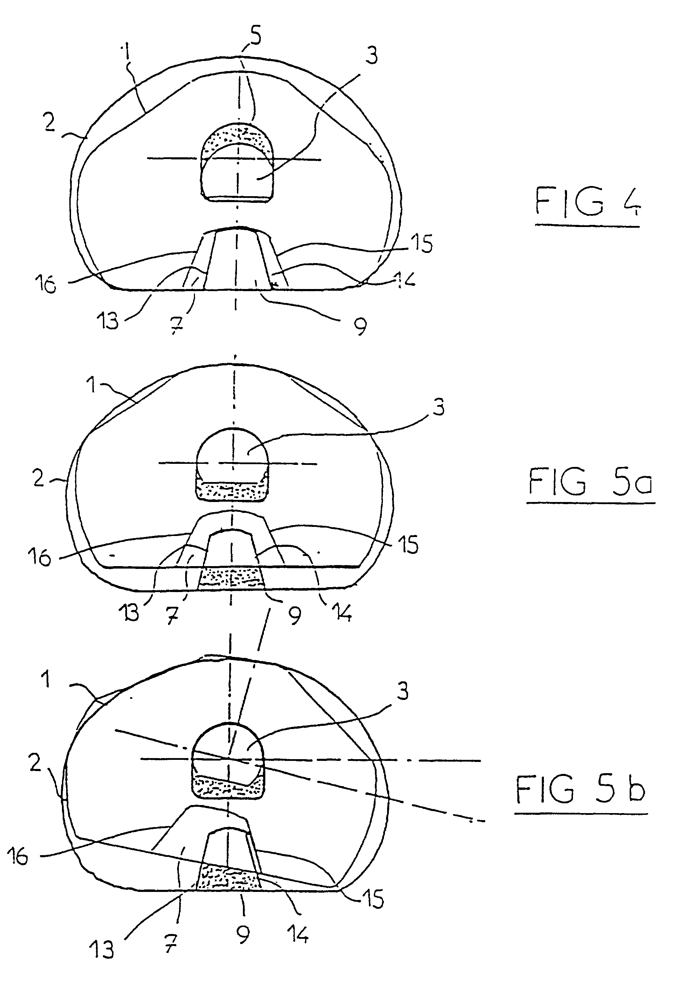

En position d'extension du genou, comme le montre la figure 4, le bossage (3) est maintenu bloqué dans la partie antérieure extrémale de l'orifice (5), interdisant ainsi tout mouvement de rotation de l'insert sur le plateau tibial.

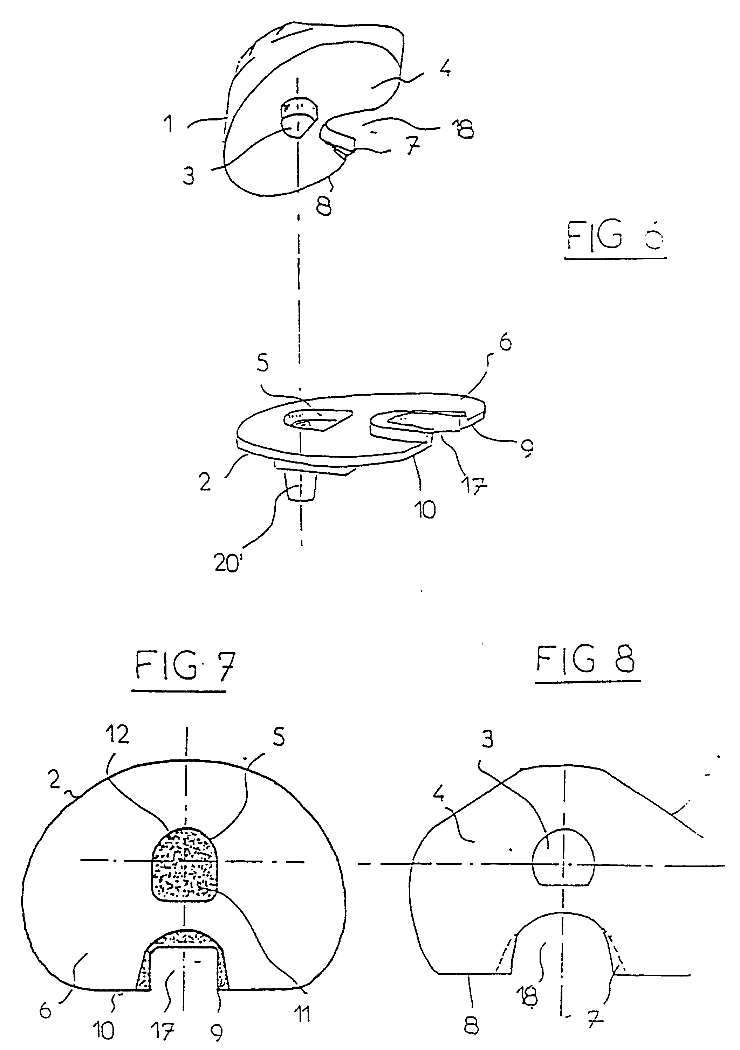

En revanche, en position fléchie, le bossage (3) est soumis à un mouvement de translation dans le sens postéro-antérieur pour venir en butée contre la face postérieure extrémale de l'ouverture (5), comme montré sur la figure 5a.

Par ailleurs, selon une autre caractéristique de l'invention, le plateau tibial (2) présente au niveau de sa bordure postérieure (10) selon l'axe médian antéro-postérieur, un épaulement en saillie (9) présentant la forme générale d'un U, dont les deux branches (13, 14) vont en s'évasant l'une de l'autre vers la bordure (10).

Comme déjà dit, cet épaulement en saillie (9) est destiné à coopérer avec une échancrure (7) ménagée sur la face inférieure de l'insert au niveau de sa bordure postérieure.

Comme le montrent les figures 2 et 3, l'épaulement en saillie (9) et l'échancrure (7) présentent une même forme générale en U, l'épaisseur et la taille selon l'axe médian antéro-postérieur de l'échancrure étant sensiblement égales à celles de l'épaulement, mais l'écartement des branches (15, 16) par rapport aux branches (13, 14) étant supérieur.

En pratique, l'écartement des branches est déterminé de sorte à ce qu'il permette de limiter la rotation de l'insert sur le plateau tibial à plus ou moins 15° par rapport à une position neutre de l'insert sur le plateau tibial, c'est à dire par rapport à un axe médian antéro-postérieur après flexion du tibia sur le fémur d'au moins 30°.

Lorsque le genou est en position d'extension, l'échancrure (7) est positionnée en butée contre l'épaulement en saillie (figure 4). A l'inverse, lors d'un mouvement de flexion, l'échancrure (7) est soumise à un mouvement de translation postéro-antérieure, dont le déplacement est égal à celui du bossage (3) dans l'orifice (5).

Lorsque le bossage se trouve en butée contre l'extrémité antérieure de l'orifice, correspondant à une flexion sensiblement de l'ordre de 30°, l'insert peut subir une rotation par rapport au plateau tibial, cette rotation étant limitée sensiblement à plus ou moins 15° par rapport à l'axe médian antéro postérieur par le contact des bras du U de l'échancrure sur les bras du U correspondant de l'épaulement en saillie.

On obtient donc un mouvement de flexion du fémur sur le tibia, au cours duquel l'insert subit d'abord un mouvement de translation postéro-antérieur puis une rotation de plus ou moins 15° par rapport au plateau tibial, permettant ainsi non seulement de reproduire l'articulation naturelle du genou, mais également de limiter l'usure de l'insert.

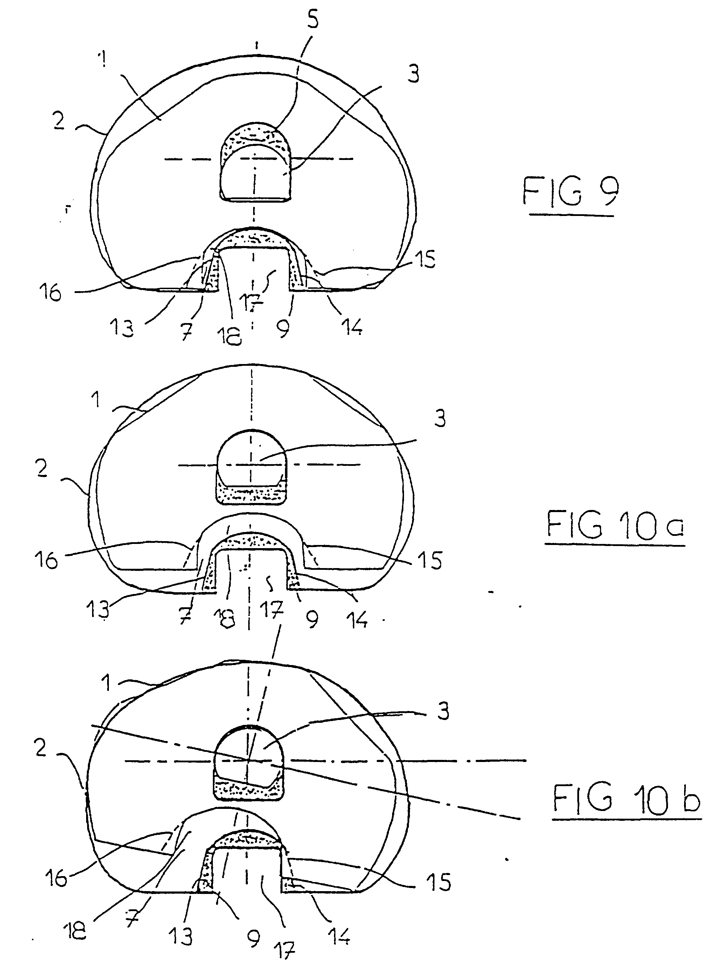

On a représenté en figures 6 à 10, une autre forme de réalisation de l'invention, plus particulièrement destinée aux prothèses de genou dans lesquelles on conserve le ligament croisé postérieur du genou. Selon cette forme de réalisation, l'épaulement (9) et l'échancrure (7) sont tous deux partiellement ajourés (17, 18), afin de permettre le passage dudit ligament.

Comme le montrent les figures, on parvient aux mêmes résultats lors d'un mouvement d'extension ou de flexion.

Les avantages ressortent bien de la description.

Ainsi, on soulignera la reproduction du mouvement naturel de l'articulation du genou selon laquelle, en position d'extension, l'insert est maintenu fixe par rapport au plateau tibial et en position de flexion, ledit insert subit un mouvement de translation antéro-postérieur et puis de rotation.

On a représenté ce mouvement de translation de l'arrière vers l'avant sur les figures 13 et 14.

En outre, les agencements prévus sur l'insert et sur le plateau tibial permettent de limiter largement l'usure de l'insert.

## Revendications

1. Prothèse de genou comprenant un plateau tibial (2) destiné à être ancré dans l'extrémité supérieure du tibia, sur lequel est rapporté un insert (1) destiné à coopérer avec une pièce fémorale destinée à être ancrée dans l'extrémité inférieure du fémur, lesdits plateau tibial (2) et insert (1) présentant des moyens complémentaires aptes à assurer :
• en position d'extension, le blocage en rotation dudit insert (1) sur le plateau tibial,
• et lors d'un mouvement de flexion, la translation dans la direction antéro-postérieur, et la rotation limitée dudit insert (1) sur le plateau tibial (2),
**caractérisée en ce que** les moyens complémentaires sont constitués :
• d'une part, d'un bossage (3) ménagé sur la face inférieure (4) de l'insert (1), en position sensiblement centrale, présentant la forme d'une portion de cylindre, ledit bossage étant destiné à coopérer avec un orifice (5) ménagé sur la face supérieure du plateau tibial (2) et présentant une partie postérieure parallélépipédique (11) et une partie antérieure hémi-cylindrique (12) de diamètre sensiblement égal à celui du bossage (3),
• et d'autre part, d'une échancrure (7) réalisée au sein de la face inférieure (4) de l'insert (1), au niveau de sa bordure postérieure (8), selon un axe médian antéro postérieur, de manière à coopérer avec un épaulement (9) en saillie ménagé sur la face supérieure (6) du plateau tibial (2).

2. Prothèse de genou suivant la revendication 1, **caractérisée en ce que** l'épaulement (9) en forme de saillie présente la forme générale d'un U, dont les deux branches (13, 14) s'évasent en direction de la bordure postérieure (10) du plateau tibial (2), l'épaulement (9) étant destiné à coopérer avec une échancrure (7) de même forme générale, ménagée sur la face inférieure (4) de l'insert (1), dont l'épaisseur et la taille selon l'axe médian antéro-postérieur sont sensiblement égales à celles de l'épaulement (9) mais dont l'écartement des branches (15, 16) est supérieur.

3. Prothèse de genou suivant la revendication 2, **caractérisée en ce que** l'épaulement en saillie (9) et l'échancrure (7) correspondante sont partiellement ajourés (17, 18).

4. Prothèse de genou suivant la revendication 2, **caractérisée en ce que** l'écartement des branches de l'échancrure est déterminé pour permettre, lors d'une flexion du tibia sur le fémur d'au moins 30°, de limiter la rotation de l'insert sur le plateau tibial à plus ou moins 15° par rapport à la position neutre de l'insert sur le plateau tibial.

## Claims

1. Knee prosthesis comprising a tibial plateau (2) which is intended to be anchored in the upper end of the tibia and on which there is attached an insert (1) which is intended to cooperate with a femoral component intended to be anchored in the lower end of the femur, the said tibial plateau (2) and the insert (1) having complementary means which are able to ensure:
• in the position of extension, the rotational blocking of the said insert (1) on the tibial plateau;
• and, during a flexion movement, the translation in the anteroposterior direction, and the limited rotation of the said insert (1) on the tibial plateau (2);
**characterized in that** the complementary means consist of:
• on the one hand, a boss (3) formed on the lower face (4) of the insert (1), in an approximately central position, having the form of a cylinder portion, the said boss being intended to cooperate with an orifice (5) formed on the upper face of the tibial plateau (2) and having a parallelepipedal posterior portion (11) and a semi-cylindrical anterior portion (12) having a diameter substantially equal to that of the boss (3);
• and, on the other hand, an indentation (7) formed within the lower face (4) of the insert (1), at its posterior edge (8), along an anteroposterior median axis in such a way as to cooperate with a projecting shoulder (9) formed on the upper face (6) of the tibial plateau (2).

2. Knee prosthesis according to Claim 1, **characterized in that** the projecting shoulder (9) has the general shape of a U, of which the two branches (13, 14) widen out in the direction of the posterior edge (10) of the tibial plateau (2), the shoulder (9) being intended to cooperate with an indentation (7) of the same general shape, formed on the lower face (4) of the insert (1), of which the thickness and size along the anteroposterior median axis are substantially equal to those of the shoulder (9), but with a greater spacing of the branches (15, 16).

3. Knee prosthesis according to Claim 2, **characterized in that** the projecting shoulder (9) and the corresponding indentation (7) are partially openworked (17, 18).

4. Knee prosthesis according to Claim 2, **characterized in that** the spacing of the branches of the indentation is determined in such a way as to make it possible, during flexion of the tibia on the femur by at least 30°, to limit the rotation of the insert on the tibial plateau to more or less 15° in relation to the neutral position of the insert on the tibial plateau.

## Patentansprüche

1. Knieprothese mit einer Schienbeinplatte (2) zum Verankern am oberen Ende des Schienbeins, an die ein Einsatz (1) angebaut ist, der mit einem Oberschenkelknochenteil zusammenwirken soll, der im unteren Ende des Oberschenkelknochens verankert werden soll, wobei die Schienbeinplatte (2) und der Einsatz (1) ergänzende Mittel aufweisen, die folgendes gewährleisten:
• in der gestreckten Haltung die Drehungsblockierung des Einsatzes (1) auf der Schienbeinplatte,
• bei einer Biegebewegung die Verschiebung in die von vorn nach hinten verlaufende Richtung und die beschränkte Drehung des Einsatzes (1) auf der Schienbeinplatte (2),
**dadurch gekennzeichnet, dass** die ergänzenden Mittel aus Folgendem bestehen:
• einerseits aus einem Höcker (3), der auf der Unterseite (4) des Einsatzes (1) in etwa mittig angeordnet ist und die Form eines zylindrischen Stücks hat, wobei der Höcker mit einer Öffnung (5) zusammenwirken soll, die auf der Oberseite der Schienbeinplatte (2) angeordnet ist und einen hinteren parallelepipedischen Teil (11) sowie einen vorderen halbzylindrischen Teil (12) mit einem Durchmesser aufweist, der in etwa dem des Höckers (3) entspricht,
• und andererseits einen Ausschnitt (7), der innerhalb der Unterseite (4) des Einsatzes (1) auf der Ebene seines hinteren Rands (8) gemäß einer von vorn nach hinten verlaufenden Mittelachse so hergestellt ist, dass er mit einem hervorstehenden Ansatz (9), der auf der Oberfläche (6) der Schienbeinplatte (2) angeordnet ist, zusammenwirkt.

2. Knieprothese nach Anspruch 1, **dadurch gekennzeichnet, dass** der hervorstehende Ansatz (9) die allgemeine Form eines U hat, dessen zwei Arme (13, 14) sich in Richtung des hinteren Rands (10) der Schienbeinplatte (2) erweitern, wobei der Ansatz (9) mit einem Ausschnitt (7) der gleichen allgemeinen Form zusammenwirken soll, der auf der Unterseite (4) des Einsatzes (1) angeordnet ist, dessen Stärke und Größe gemäß der von vorn nach hinten verlaufenden mittleren Achse in etwa gleich denen des Ansatzes (9) sind, dessen Abstand der Arme (15, 16) jedoch größer ist.

3. Knieprothese nach Anspruch 2, **dadurch gekennzeichnet, dass** der hervorstehende Ansatz (9) und der entsprechende Ausschnitt (7) teilweise durchbohrt (17, 18) sind.

4. Knieprothese nach Anspruch 2, **dadurch gekennzeichnet, dass** der Abstand der Arme des Ausschnitts so festgelegt ist, dass er beim Biegen des Schienbeins um mindestens 30° auf dem Oberschenkelknochen die Drehung des Einsatzes auf der Schienbeinplatte um mehr oder weniger 15° in Bezug auf die neutrale Position des Einsatzes auf der Schienbeinplatte beschränkt.
